# EUROPÄISCHE PATENTSCHRIFT

(11) **EP 1 492 572 B1**
(45) Veröffentlichungstag und Bekanntmachung des Hinweises auf die Patenterteilung: **03.08.2005**
(21) Anmeldenummer: 03711702.5
(22) Anmeldetag: 28.03.2003
(51) Int. Cl.: A61L 2/04

(54) **ANLAGE ZUM STERILISIEREN, PASTEURISIEREN UND/ODER DESINFIZIEREN PUMP- ODER RIESELFAEHIGER MEDIEN**
INSTALLATION FOR STERILIZING, PASTEURIZING, AND/OR DISINFECTING PUMPABLE OR POURABLE MEDIA
INSTALLATION DESTINEE A LA STERILISATION, PASTEURISATION ET/OU DESINFECTION DE MILIEUX POUVANT ETRE POMPES OU SUSCEPTIBLES D'ECOULEMENT

(30) Priorität: 28.03.2002 AT 4942002
(43) Veröffentlichungstag der Anmeldung: 05.01.2005
(73) Patentinhaber: Katschnig, Helmut, Dr., A-8750 Judenburg Steiermark (AT)
(72) Erfinder: KATSCHNIG, Helmut, A-8750 Judenburg (AT); GRUBER, Ernst, A-8750 Judenburg (AT)
(74) Vertreter: Itze, Peter, Dipl.-Ing.
(86) Internationale Anmeldenummer: PCT/AT2003/000089
(87) Internationale Veröffentlichungsnummer: WO 2003/082354

(56) Entgegenhaltungen:
- US-A- 3 579 631
- US-A- 5 403 564
- US-A- 5 863 580

## Beschreibung

Die Erfindung bezieht sich auf eine Anlage zum Sterilisieren, Pasteurisieren und/oder Desinfizieren pump- oder rieselfähiger Medien, bei welcher wenigstens eine Heizquelle und eine mittels Thermofühler überwachte Temperaturhaltestrecke sowie ein Ablauf vorgesehen ist, und bei welcher der zur Behandlung erforderliche Druck durch wenigstens ein gegen ein Gegendruckorgan förderndes Förderaggregat in der Temperaturhaltestrecke aufrechterhalten ist, wobei an die von der Temperaturhaltestrecke wegführende Leitung zwei Ablaufleitungen angeschlossen sind, dadurch gekennzeichnet, daß in jede Ablaufleitung je ein Gegendruckorgan und ein Abschlußorgan eingebaut sind, wobei die eine Leitung in den Abfluß mündet und die andere Leitung als Rückführleitung vor der Heizquelle zur Zufuhrleitung führt.

Aus AT 399 658 B geht eine Anlage hervor, bei welcher nach dem durch ein zwangsfördernden Förderaggregat gebildetes Gegendruckorgan und vor Einmündung in den Abfluß ein Dreiweg-Ventil eingeschaltet ist, von welchem eine Rückführleitung zum Zulauf abzweigt. Eine solche Ausbildung hat den Nachteil, daß dadurch, daß in der von der Temperaturhaltestrecke wegführenden Leitung je nach Verfahrensstadium, entweder kontaminiertes oder bereits pasteurisiertes, desinfiziertes oder sterilisiertes Medium fließt, bei Betätigung des Dreiweg-Ventiles, also bei Umschalten des Mediumflußes von der Rückführung zum Abfluß, in Leitungsstücken oder in der Pumpe bzw. dem Umschaltorgan verbliebenes oder kontaminiertes bzw. ungenügend behandeltes Medium in den Abfluß gelangen kann.

Gemäß US 5,863,580 ist nach einer Temperaturhaltestrecke ein Verteilerventil vorgesehen, das je nach Temperatur des ankommenden Materials dieses entweder zum Abkühlen zu einem Wärmetauscher oder erneut in den Behandlungskreislauf umleitet. Das Verfahren läuft unter Normaldruck ab und kann daher Sterilisationstemperaturen nicht erreichen.

Um einen gezielten Druckabbau unter gleichzeitiger Vermeidung ungezielter Dampfbildung und auch die oben angeführte Möglichkeit des Eindringens von kontaminiertem Material in den Abfluß auszuschalten, sind erfindungsgemäß an die von der Temperaturhaltestrecke wegführenden Leitung zwei Ablaufleitungen angeschlossen, die je ein Gegendruckorgan und ein Abschlußorgan eingebaut haben, wobei die eine Leitung in den Abfluß mündet und die andere Leitung als Rückführleitung vor der Heizquelle zur Zufuhrleitung führt, und daß ein Wärmetauscher vorgesehen ist, in dem das von der Temperaturhaltestrecke kommende Medium in den Ablaufleitungen (13, 14) in Gegenstrom zu dem zur Heizquelle strömenden Medium geführt ist.

Vorteilhafterweise können in dem Wärmetauscher die Bereiche der beiden Ablaufleitungen durch Wärmetauschschlangen gebildet sein, die einen geringeren Durchflußquerschnitt als die von dem Wärmetauscher vorgesehenen Leitungsteile besitzen.

Weiters kann in die zur Temperaturhaltestrecke führende Leitung ein Durchflußmesser eingebaut sein, womit ermöglicht ist, die Anlage auch mit unterschiedlichen Durchflußgeschwindigkeiten so zu betreiben, daß innerhalb der Temperaturhalteshecke die erforderliche Verweilzeit des Mediums auf der gewünschten Temperatur eingehalten werden kann, was insbesondere dann zum Tragen kommt, wenn unterschiedliche Prozesse, also Sterilisieren, Pasteurisieren oder Desinfizieren durchgeführt werden. Weiters kann in der in den Abfluß mündenden Leitung nach dem Gegendruckorgan ein weiterer Temperaturfühler vorgesehen sein, um zu überwachen, daß die Temperatur des in den Abfluß gelangenden Mediums 60° C nicht übersteigt, da bei höheren Temperaturen die Abfluß- bzw. Kanalleitungen Schaden nehmen können. Schließlich kann in die Leitung zwischen zwangsförderndem Förderorgan und Gegendruckorgan ein Drucksensor eingebaut werden, womit eine zusätzliche Überwachungsmöglichkeit der Anlage gegeben ist.

In der Zeichnung sind Schemata des Aufbaus des Erfindungsgegenstandes wiedergegeben.

Fig. 1 zeigt eine erste Ausführungsvariante mit Förderorganen als Gegendruckorgane und

Fig. 2 eine Variante mit statischen Gegendruckorganen.

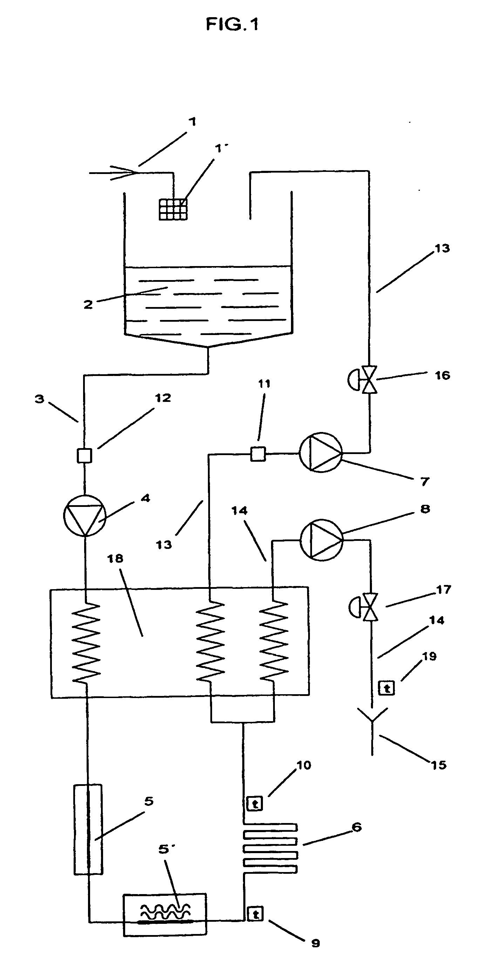

Beim Ausführungsbeispiel gemäß Fig. 1 ist mit 1 ein Zulauf für kontaminiertes Medium bezeichnet, welcher über einen Filter 1' in einem Vorratsbehälter 2 einmündet. Von diesem Vorratsbehälter führt eine Zufuhrleitung 3 zu einer Pumpe 4, welche ein zwangsfördemdes Förderorgan bildet. Von dieser Pumpe 4 wird das zu behandelnde Medium über eine Heizung 5 bzw. 5' zu einer Temperaturhaltestrecke 6 geführt. Bei 5 ist die Heizung allgemein angedeutet, die jede passende Bauart und Energiequelle aufweisen kann. Zusätzlich oder alternativ kann eine Mikrowellenheizquelle 5' vorgesehen sein. Die von der Temperaturhaltestrecke 6 wegführende Leitung teilt sich in zwei Leitungen 13 und 14, wobei die Leitung 13 zurück zum Vorratsbehälter 2 und die Leitung 14 zum Abfluß 15 führt. In die zurückführende Leitung 13 und die zum Abfluß führende Leitung 14 ist je eine Pumpe 7, 8 eingeschaltet, welche ebenfalls ein, vorzugsweise zwangsförderndes, Förderaggregat bildet. Der Temperaturhaltestrecke 6 ist ein Wärmefühler 9 vorgeschaltet und ein weiterer Wärmefühler 10 nachgeschaltet. Mittels dieser beiden Wärmefühler wird die Temperaturkonstanthaltung über die Temperaturhaltestrecke 6 überwacht. In der Leitung 13 ist vor der Pumpe 7 ein Drucksensor 11 vorgesehen, mittels welchem der Druck innerhalb des Bereiches zwischen der Pumpe 4 und der Pumpe 7 bzw. 8 gemessen wird. In die zum Vorratsbehälter 2 führende Leitung 13 ist ein Absperrorgan 16 und in die zum Abfluß 15 führende Leitung 14 ein Absperrorgan 17, jeweils der Pumpe nachgeschaltet, eingebaut. Bei selbstsperrenden Pumpen als Gegendruckorgan könnten die Absperrorgane 16, 17 entfallen. Mit 18 ist ein Wärmetauscher bezeichnet, mit welchem das der Heizung 5 bzw. 5' zugeführte zu behandelnde Medium durch das von der Wärmehalteschlange 6 abgeführte Medium vorgeheizt wird.

Der Pumpe 4 vorgeschaltet ist ein Durchflußmesser 12, der dazu dient, die aktuelle Durchflußmenge zwecks Errechnung der Verweilzeit innerhalb der Temperaturhaltestrecke zu überwachen.

Zum Anfahren der Anlage wird das zu behandelte Medium durch die Pumpe 4, die Heizung 5 oder den Mikrowellengenerator 5', wobei, wie angeführt, auch beide gleichzeitig vorhanden sein können, über die Temperaturhaltestrecke 6, die Leitung 13, die Pumpe 7 und das Absperrorgan 16 im Kreislauf geführt. Die Pumpe 8 in die Leitung 14 ist abgeschaltet und das Absperrorgan 17 ist geschlossen, so daß über diese Leitung auch nicht im Bereich vor der Pumpe 8 ein Flüssigkeits- oder Mediumstrom erfolgen kann. In diesem Zustand wird die Anlage solange betrieben, bis der der Temperaturhaltestrecke 6 vorgeschaltete Temperaturfühler 9 und der der Temperaturhaltestrecke nachgeschaltete Temperaturfühler 10 konstant die gewünschte Temperatur zeigen, wobei über die vom Durchflußmesser 12 gemessene Menge die Verweilzeit des zu behandelnden Mediums in der Temperaturhaltestrecke ermittelbar und durch die Einstellungen der Pumpe 4 und 7 auf den gewünschten Wert eingeregelt ist. Sobald die gewünschte Temperaturkonstanz und Verweilzeit erreicht ist, wird die in der Rückführleitung 13 angeordnete Pumpe 7 abgeschaltet und dafür die in der zum Abfluß 15 führenden Leitung 14 vorgesehen Pumpe 8 eingeschaltet und die jeweiligen zugehörigen Ventile 16, 17 entsprechend umgeschaltet d.h., daß das Absperrorgan 16 geschlossen und das Absperrorgan 17 geöffnet wird. Der Drucksensor 11 bleibt in beiden Betriebszuständen mit dem im System herrschenden Druck beaufschlagt, so daß auch über diesen Sensor zusätzlich immer der für die Einhaltung der Betriebsparameter erforderliche Druck überwacht werden kann. In der in den Abfluß führenden Leitung 14 ist unmittelbar vor deren Mündung ein weiterer Temperaturfühler 19 angeordnet, mittels welchem die Temperatur des abfließendem Mediums überwacht wird. Steigt die Temperatur am Messfühler 19 über 60° C, dann wird entweder das Gerät abgeschaltet oder auf Umlauf geschaltet, bzw. es wird ein zusätzlicher, nicht dargestellter Wärmetauscher aktiviert, um sicherzustellen, daß der Ablauf nicht zu heiß erfolgt.

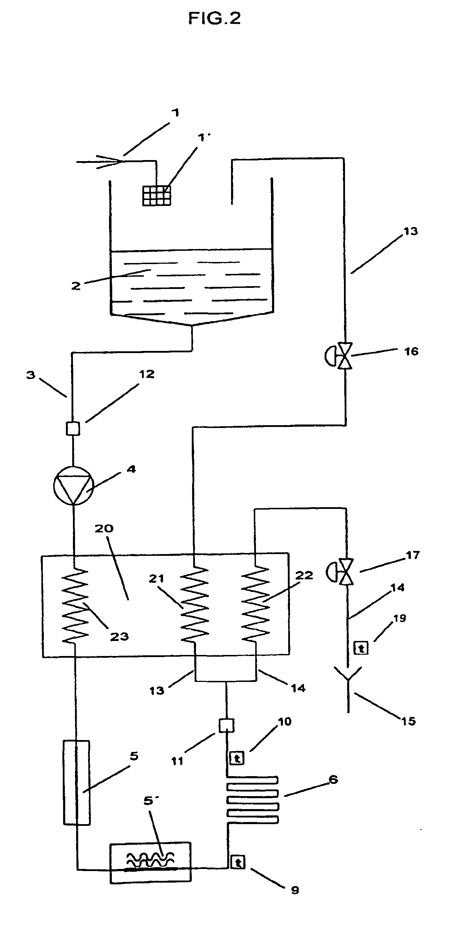

Bei der Ausführungsvariante gemäß Fig. 2 sind für gleiche Anlagenteile die gleichen Bezugszeichen wie in Fig. 1 verwendet. So ist mit 1 der Zulauf, mit 1' ein Filter, mit 2 ein Vorratsbehälter, mit 3 eine Zufuhrleitung und mit 4 eine Druck aufbauende Pumpe bezeichnet. Mit 5 ist ein herkömmliches Heizorgan und mit 5' ein Mikrowellenheizorgan bezeichnet, wobei wie auch bei der Ausführung gemäß Fig. 1 angeführt, entweder das herkömmliche Heizorgan 5 oder die Mikrowellenheizung 5' allein oder aber beide gemeinsam vorgesehen sein können. Der Heizung 5 bzw. 5' nachgeschaltet ist eine Temperaturhaltestrecke 6, die mittels Temperaturfühler 9, 10, von welchen einer der Temperaturhaltestrecke 6 vorgeschaltet und der andere dieser nachgeschaltet ist, überwacht. Der Temperaturhaltestrecke 6 nachgeschaltet ist in der Leitung 13 ein Drucksensor 11 und in der Leitung 3 ein Durchflußmesser 12 zur Überwachung der Anlage eingebaut.

Die von der Temperaturhaltestrecke 6 wegführende Leitung ist in zwei Äste 13, 14 aufgeteilt, von denen der eine, 13, zum Vorratsbehälter 2 zurückführt und der andere Ast, 14, in einem Abfluß 15 einmündet. In beiden Leitungsästen 13 bzw. 14 sind Abschlußorgane 16 bzw. 17 vorgesehen. Vor der Mündung des Leitungsastes 14 in den Abfluß 15 ist der zusätzlicher Temperaturmessfühler 19 vorgesehen.

Zur Erzeugung des Gegendruckes ist bei der Ausbildung gemäß Fig. 2 im Gegensatz zu der Ausbildung gemäß Fig. 1, ein spezieller den Durchfluß begrenzender Wärmetauscher 20 vorgesehen, in welchem die Bereiche der beiden Leitungsäste 13, 14 durch Wärmetauschschlangen 21, 22 gebildet sind, die geringeren Durchflußquerschnitt als die vor dem Wärmetauscher vorgesehenen Leitungsteile besitzen. Sowohl die Wärmetauschschlange 21 als auch die Wärmetauschschlange 22 sind im Gegenstrom zum Wärmetauschbereich 23 der Leitung 3 geführt. Diese Bereich 23 weist keinerlei Durchflußbeschränkungen im Bezug auf die Leitung 3 auf.

Bei Betrieb der Vorrichtung wird die Flüssigkeit aus dem Vorratsbehälter 2 über die Leitung 3 durch die Pumpe 4 der Heizung 5 bzw. 5' zugeführt, wobei wie schon angeführt entweder eine der beiden Heizungen oder beide gemeinsam vorgesehen sein können. Nach dem Aufheizen gelangt das zu behandelte Medium in die Temperaturhaltestrecke 6, wobei durch die Ausbildung der Wärmetauscherschlangen 21, 22 ein gegen den Förderdruck der Pumpe 4 wirkende Druck erzeugt ist. Dieser wird mittels des Drucksensors 11 überwacht. Zum Anfahren der Anlage ist das Ventil 17 in der zum Abfluß 15 führenden Leitung 14 geschlossen und das Ventil 16 in der zum Vorratsbehälter 2 zurückführenden Leitung 13 geöffnet. Es wird nun so lange Flüssigkeit im Kreislauf geführt, bis auf Grund des Aufheizens durch die Heizung 5, 5', in der Temperaturhaltestrecke 6 die am Temperaturfühler 10 gemessene Temperatur und der am Drucksensor 11 gemessene Druck jene Werte erreicht hat, die sicherstellen, daß das aus der Temperaturhaltestrecke 6 abfließende Medium zuverlässig sterilisiert, pasteurisiert oder desinfiziert ist. Danach wird das Ventil 16 der Leitung 13 geschlossen und das Ventil 17 der Leitung 14 geöffnet. Es erfolgt dann das Ableiten des behandelten und sterilisierten Flüssigkeit bzw. des Mediums in den Abfluß.

Die Temperatur des in den Abfluß gelangenden Mediums wird mittels des Messfühlers 19 überwacht um sicherzustellen, daß der Ablauf nicht zu heiß erfolgt, da wir schon angeführt sonst eine Beschädigung des Kanalsystems auftreten kann.

In nicht dargestellter Weise könnte auf den der Temperaturhaltestrecke vorgeschalteten Thermofühler 9 verzichtet werden, wenn auf Grund der im Thermofühler 10 ermittelten Temperatur, gegebenenfalls über den am Drucksensor 11 gemessenen Druck, und der über den Durchflußmesser 12 ermittelten Durchflußmenge eine ausreichende Überwachung der Behandlungstemperatur und -dauer vorgenommen wird.

## Patentansprüche

1. Anlage zum Sterilisieren, Pasteurisieren und/oder Desinfizieren pump- oder rieselfähiger Medien, bei welcher wenigstens eine Heizquelle und eine mittels Thermofühler überwachte Temperaturhaltestrecke sowie ein Ablauf vorgesehen ist, und bei welcher der zur Behandlung erforderliche Druck durch wenigstens ein gegen ein Gegendruckorgan förderndes Förderorgan der Anlage in der Temperaturhaltestrecke aufrechterhalten ist, wobei an die von der Temperaturhaltestrecke wegführende Leitung zwei Ablaufleitungen angeschlossen sind, **dadurch gekennzeichnet, daß** in jede Ablaufleitung (13, 14) je ein Gegendruckorgan (7, 8 21, 22) und ein Abschlußorgan (16, 17) eingebaut sind, wobei die eine Leitung (14) in den Abfluß (15) mündet und die andere Leitung (13) als Rückführleitung vor der Heizquelle (5 bzw. 5') zur Zufuhrleitung (3) führt, und daß ein Wärmetauscher (20) vorgesehen ist, in dem das von der Temperaturhaltestrecke (6) kommende Medium in den Ablaufleitungen (13, 14) in Gegenstrom zu dem zur Heizquelle (5,5') strömenden Medium geführt ist.

2. Anlage nach Anspruch 1, **dadurch gekennzeichnet, daß** in dem Wärmetauscher (20) die Bereiche der beiden Ablaufleitungen (13 bzw. 14) durch Wärmetauschschlangen (21 bzw. 22) gebildet sind, die geringeren Durchflußquerschnitt als die vor dem Wärmetauscher vorgesehenen Leitungsteile besitzen.

3. Anlage nach Anspruch 1 oder 2, **dadurch gekennzeichnet, daß** in die zur Temperaturhaltestrecke (6) führende Leitung (3) ein Durchflußmesser (12) eingebaut ist.

4. Anlage nach einem der Ansprüche 1 bis 3, **dadurch gekennzeichnet, daß** in der in den Abfluß (15) mündenden Leitung (14) nach dem Gegendruckorgan (8) ein weiterer Temperaturfühler (19) vorgesehen ist.

5. Anlage nach einem der Ansprüche 1 bis 4, **dadurch gekennzeichnet, daß** in die Leitung (13) zwischen zwanasförderndem Förderorgan (4) und Gegendruckorgan (7,8 bzw. 21,22) ein Drucksensor (11) eingebaut ist.

## Claims

1. Apparatus for sterilising, pasteurising and/or disinfecting pumpable or flowable media, wherein at least one heat source and a temperature maintaining zone monitored by thermosensors and an outflow are provided, and wherein the pressure required for the treatment is maintained in the temperature maintaining zone by at least one conveying member of the apparatus which conveys counter to a counter-pressure member, two outlet lines being connected to the line leading away from the temperature maintaining zone, **characterised in that** a counter-pressure member (7, 8, 21, 22) and a closure member (16, 17) is mounted in each outlet line (13, 14), one line (14) opening into the outflow (15) and the other line (13) acting as a return line leading to the feed line (3) upstream of the heat source (5 or 5'), and **in that** a heat exchanger (20) is provided in which the medium coming from the temperature maintaining zone (6) is carried in the outlet lines (13, 14) in countercurrent to the medium flowing to the heat source (5, 5').

2. Apparatus according to claim 1, **characterised in that** in the heat exchanger (20) the regions of the two outlet lines (13 and 14) are formed by heat exchange coils (21 and 22, respectively) which have a smaller cross-section of flow than the sections of line provided upstream of the heat exchanger.

3. Apparatus according to claim 1 or 2, **characterised in that** a flow gauge (12) is incorporated in the line (3) leading to the temperature maintaining zone (6).

4. Apparatus according to one of claims 1 to 3, **characterised in that** another temperature sensor (19) is provided in the line (14) opening into the outflow (15), downstream of the counter-pressure member (8).

5. Apparatus according to one of claims 1 to 4, **characterised in that** a pressure sensor (11) is incorporated in the line (13) between the forced-flow conveying member (4) and the counter-pressure member (7, 8 or 21, 22).

## Revendications

1. Installation destinée à la stérilisation, la pasteurisation et/ou la désinfection de milieux pouvant être pompés ou susceptibles d'écoulement, dans laquelle sont prévus au moins une source de chaleur et un parcours de maintien en température, contrôlé par des détecteurs de température, ainsi que des moyens d'écoulement, et dans laquelle la pression requise pour le traitement est maintenue, dans le parcours de maintien en température, par au moins un organe d'entraînement de l'installation, agissant à l'encontre d'un organe de contre-pression, deux conduits de vidange étant raccordés à la conduite qui part du parcours de maintien en température, **caractérisée en ce que** dans chaque conduit de vidange (13, 14) sont montés un organe de contre-pression (7, 8, 21, 22) et un organe d'obturation (16, 17), l'un des conduits (14) débouchant dans le dispositif d'écoulement (15) et l'autre conduit (13) menant, en tant que conduit de recyclage, de la source de chaleur (5 ou 5') au conduit d'alimentation (3), et **en ce qu'**est prévu un échangeur de chaleur (20), dans lequel le milieu qui provient du parcours de maintien en température (6), présent dans les conduits de vidange (13, 14) est dirigé à contre-courant du milieu qui s'écoule en direction de la source de chaleur (5, 5').

2. Installation selon la revendication 1, **caractérisée en ce qu'**à l'intérieur de l'échangeur de chaleur (20), les zones des deux conduits de vidange (respectivement 13 et 14) sont constituées par des serpentins échangeurs de chaleur (21 et/ou 22) qui présentent une section transversale d'écoulement inférieure à celle des parties de conduits qui sont prévus en amont de l'échangeur de chaleur.

3. Installation selon la revendication 1 ou 2, **caractérisée en ce qu'**un débitmètre (12) est monté dans le conduit (3) qui mène au parcours de maintien en température (6).

4. Installation selon l'une des revendications 1 à 3, **caractérisée en ce qu'**est prévu dans le conduit (14) qui débouche dans le dispositif d'écoulement (15), en aval de l'organe de contre-pression (8), un autre détecteur de température (19).

5. Installation selon l'une des revendications 1 à 4, **caractérisée en ce qu'**un capteur de pression (11) est monté dans le conduit (13) entre l'organe d'entraînement (4) qui exerce un entraînement forcé et l'organe de contre-pression (respectivement 7, 8 ou 21, 22),.
